# EUROPEAN PATENT SPECIFICATION

(11) **EP 4 247 339 B1**
(45) Date of publication and mention of the grant of the patent: **28.10.2026**
(21) Application number: 21901175.6
(22) Date of filing: 01.12.2021
(51) Int. Cl.: A61K 9/00, A61K 9/127, A61K 31/12, A61P 35/00, A61K 31/17, A61K 31/704

(54) **TARGETED DRUG DELIVERY SYSTEM WITH CURCUMIN SUPPLEMENT IN THE TREATMENT OF GLIOBLASTOMA**
GEZIELTES ARZNEIMITTELABGABESYSTEM MIT CURCUMINZUSATZ BEI DER BEHANDLUNG VON GLIOBLASTOM
SYSTÈME D'ADMINISTRATION CIBLÉE DE MÉDICAMENT COMPORTANT UN SUPPLÉMENT DE CURCUMINE DANS LE TRAITEMENT DU GLIOBLASTOME

(30) Priority: 01.12.2020 TR 202019429; 30.11.2021 TR 202118737
(43) Date of publication of application: 27.09.2023
(73) Proprietor: Ege Universitesi, 35100 Izmir (TR)
(72) Inventor: SANLIER, Senay, 35040 Bornova/ Izmir (TR); AK DEMIROZ, Güliz, 35040 Bornova/ Izmir (TR); KARADAG, Belgüzar, 35100 Bornova/ Izmir (TR); KARAKAYALI, Tugba, Bölümü/Argefar, Izmir (TR)
(74) Representative: Potter Clarkson
(86) International application number: PCT/TR2021/051321
(87) International publication number: WO 2022/119539

(56) References cited:
- EP-A1- 3 711 770
- WO-A1-2020/225769
- LI JIE, ZHAO JIAQIAN, TAN TIANTIAN, LIU MENGMENG, ZENG ZHAOWU, ZENG YIYING, ZHANG LELE, FU CHAOMEI, CHEN DAJING, XIE TIAN: "Nanoparticle Drug Delivery System for Glioma and Its Efficacy Improvement Strategies: A Comprehensive Review", INTERNATIONAL JOURNAL OF NANOMEDICINE, vol. Volume 15, pages 2563 - 2582, XP055941984, DOI: 10.2147/IJN.S243223
- SESARMAN ALINA; TEFAS LUCIA; SYLVESTER BIANCA; LICARETE EMILIA; RAUCA VALENTIN; LUPUT LAVINIA; PATRAS LAURA; PORAV SEBASTIAN; BANC: "Co-delivery of curcumin and doxorubicin in PEGylated liposomes favored the antineoplastic C26 murine colon carcinoma microenvironment", DRUG DELIVERY AND TRANSLATIONAL RESEARCH, SPRINGER, GERMANY, vol. 9, no. 1, 12 November 2018 (2018-11-12), Germany , pages 260 - 272, XP036669488, ISSN: 2190-393X, DOI: 10.1007/s13346-018-00598-8
- WEI MINYAN, GUO XIUCAI, TU LIUXIAO, ZOU QI, LI QI, TANG CHENYI, CHEN BAO, WU CHUANBIN, WEI MINYAN: "Lactoferrin-modified PEGylated liposomes loaded with doxorubicin for targeting delivery to hepatocellular carcinoma", INTERNATIONAL JOURNAL OF NANOMEDICINE, vol. 2015, 1 January 2015 (2015-01-01), pages 5123 - 5137, XP055942007, DOI: 10.2147/IJN.S87011

## Description

### Technical Field Related to the Invention

The invention relates to liposomes containing doxorubicin (DOX), carmustine (BCNU), and curcumin (CUR) developed for use in the treatment of glioblastoma.

### State of the Art of the Invention (Prior Art)

Glioblastoma multiform is one of the most aggressive and common primary brain tumors that occur in the central nervous system. It is more common in the 55-60 age range even though it is observed in all ages and ranks third among the cancers that cause death in the 15-34 age range. Glioblastoma is seen in 17% of 0.1 million people diagnosed with central nervous system tumors and primary brain tumors every year. The most frequent type of cancer after leukemia is brain tumor in individuals between the ages of 0 and 19 according to the statement of the American Union of Brain Tumors. In addition, the most common cause of death after leukemia in individuals between the ages of 1 and 19 is brain tumors. Meningiomas rank first among brain tumors with a rate of 36.4%. This is followed by glioma with a rate of 27%. In addition, gliomas constitute 80% of malignant brain tumors. Glioblastoma, which is a grade IV glioma, is very aggressive and has the potential to spread very quickly and is seen approximately four times more than grade III anaplastic astrocytoma. Glioblastoma is the most common and destructive form of primary brain tumor. Glioblastomas should be treated as soon as possible as they spread very quickly. The mean survival time of patients with glioblastoma with standard of treatment is approximately 11-15 months. Therefore, it is important to develop new treatment approaches for glioblastoma. There is no definitive treatment for glioblastoma multiforme (GB) even though some progress has been made in diagnosis and treatment over the years. Accordingly, drugs are used separately in the treatment of glioblastoma. Among the treatment options, the first step in the treatment of glioblastoma (GB) is the surgical procedure to remove the tumor. Methods such as radiotherapy and chemotherapy are applied in the next step. However, the survival rate of the patients is stated as approximately 14 months. The most commonly used anti-cancer agent in treatment is carmustine, which is used as primary or adjuvant chemotherapy. It has an average survival time of 14.6 months from diagnosis as a result of this treatment.

Glioblastoma multiform, which is the most common primary brain tumor, constitutes approximately 40% of malignant brain tumors, as well as limited treatment options. They consist predominantly of abnormal astrocytic cells, but also of different cell types and areas of necrotic cells, so they are difficult to treat. A few of the factors limiting the treatment are the inability to accumulate the drug in sufficient concentration in the tumor area, the prevention of the drug from passing through the blood brain barrier, and the side effects caused by the chemotherapeutic agent. The disadvantages of treatment methods necessitated the development of effective strategies. Therefore, it is important to develop new treatment approaches for glioblastoma, which covers a very high part of malignant brain tumors.

Recent studies have aimed to make the drug effective in the targeted area, to show the therapeutic effect in a shorter time, and to reduce its side effects since many drugs in the market are ineffective in the treatment of brain diseases because they cannot reach the brain by passing the blood brain barrier. The use of liposomes is advantageous in order to reduce the side effects of drugs, to ensure the passage through the blood brain barrier and to act on the desired area of the drug through active targeting. Liposomal curcumin or curcuminoid with at least one of chemotherapeutic agent (temozolomide, etoposide, doxorubicin, cisplatin, paclitaxel, carmustine, lomustine, ceramide and phosphorylcholine) which is synergistic with carcumin was used intravenously for glioblastoma treatment described in EP 3711770 A1 entitled *"A combination of a curcuminoid, an empty liposome and a chemotherapeutic agent for use in treatment of glioblastoma".* The ease of preparation of the selected drug delivery system and its contribution to the reduction of treatment costs increase its usability in clinical applications. Drugs are targeted directly to the Central Nervous System (CNS), reducing systemic exposure and accordingly undesirable side effects with intranasal administration. Administration to the CNS through the nose occurs within a few minutes along both the olfactory and trigeminal nerve pathways along an extracellular pathway and does not require medication to bind to any receptor or axonal delivery.

There are nanocarrier systems in which doxorubicin, carmustine and curcumin active substances are individually or in binary combination in the state of the literature. It is also known that there are different drug delivery systems containing BCNU for the treatment of glioblastoma and that BCNU, DOX, and Curcumin drugs are used in separate drug delivery systems. However, a single carrier system containing three active substances, doxorubicin, carmustine and curcumin is not known in the literature.

### Brief Description and Objects of the Invention

The present invention relates to liposomes containing doxorubicin (DOX), carmustine (BCNU), and curcumin (CUR) developed for use in the treatment of glioblastoma, which meets the aforementioned needs, eliminates all disadvantages, and provides some additional advantages.

The primary object of the invention is to reduce the negativities observed in chemotherapy in the treatment of glioblastoma cancer, to increase drug delivery to cellular levels, to provide effective and safe treatment at low doses, to minimize the toxic properties of drugs, to obtain the desired level of pharmacological response in the target region without damaging healthy tissues.

The invention has been provided to direct the triple drug combination with liposome structure to the targeted drug carrier system in low doses. In addition, the drug delivery system prepared will be used intranasally. In order for this system to pass the Blood Brain Barrier (BBB), it is aimed that the surface of the triple drug system in low concentrations bound to lipids will be covered with the PEG system and pass this barrier. The use of PEGylated liposomes promotes the accumulation of the encapsulated drug in the central nervous system (CNS). This will allow the system to go directly to the targeted cancer cell in the treatment of glioblastoma and will not damage the intact tissues and cells in any way. Other treatment methods can cause damage to intact tissue cells. Meanwhile, patients wear out mentally during this difficult treatment process. The treatment process will be directly directed to the targeted cancer area in the invention. While the drugs used orally in the treatment can have a side effect on the whole body, the final formulation of the liposome containing doxorubicin, carmustine and curcumin coated with PEG in the invention will be administered intranasally to mice in in vivo studies, so it will be ensured that it will go directly to the target area.

Carmustine, one of the three drugs used in the invention (doxorubicin, carmustine, and curcumin), is in the alkylating agent class and is chemotherapeutic for glioblastoma patients and causes apoptosis by causing DNA damage. It has many side effects such as pulmonary toxicity, nausea, vomiting, dizziness, and loss of coordination. Doxorubicin is used for therapeutic purposes in many types of cancer, including glioblastoma. It shows its anticancer effect by causing cell death through intercalation in DNA The most commonly used treatment is carmustine, which can be taken as primary or adjuvant chemotherapy. This treatment results in a current prognosis for an average survival of 14.6 months from diagnosis. Curcumin, on the other hand, is a plant-derived agent with various therapeutic effects. Curcumin can be used alone or in combination with other chemotherapeutic agents.

Liposomes are preferred due to their biocompatibility, low toxicity, and functionalization of the surfaces in the invention. Liposomes will be reduced in size with the help of a microfluidic device with the addition of a triple drug system after the pre-emulsion formation step and their surfaces will be covered with DSPE-mPEG2000 (ammonium salt). The effectiveness of liposomes containing the prepared combined drug system in the brain cancer cell line will be examined. In addition, the potential of the liposomal structure to be used in the treatment of glioblastoma (GB) will be investigated in vivo and intranasal administration will be performed. Intacerebral implantation of tumor cells using nude mice will be performed and the cancer treatment potential of the system and survival in mice will be monitored, following the biodistribution and pharmacokinetic studies of the drug delivery system.

### Definitions of Figures Describing the Invention

**Figure 1****:** Hydrodynamic size and PDI value of the empty liposome obtained as a result of the flow 15 at 137895 Pa (20.000 psi) in the microfluidic device
**Figure 2****:** Zeta potential analysis of the empty liposome obtained as a result of the flow 15 at 137895 Pa (20.000 psi) in the microfluidic device
**Figure 3****:** SEM analysis image of the empty liposome collected at the flow 15 at 137895 Pa (20.000 psi) pressure in the microfluidic device
**Figure 4****:** TEM analysis images of the empty liposome prepared under optimum conditions
**Figure 5****:** Hydrodynamic size distribution graph of triple drug-containing liposomes prepared with BCNU, CUR, and DOX at initial concentrations of 10 µg/mL
**Figure 6****:** Potential graph of triple drug-containing liposomes prepared with BCNU, CUR, and DOX at initial concentrations of 10 µg/mL.

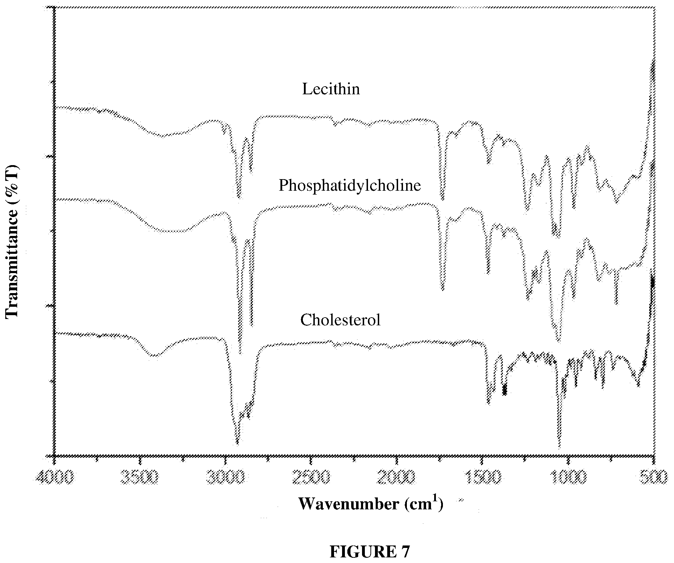

**Figure 7****:** The FTIR spectrum of Lecithin, phosphatidylcholine and lecithin in the structure of the liposome.
**Figure 8****:** The FTIR spectrum of curcumin.
**Figure 9****:** The FTIR spectrum of doxorubicin.
**Figure 10****:** The FTIR spectrum of DSPE-mPEG2000.
**Figure 11****:** The FTIR spectrum of the empty liposome.
**Figure 12****:** The FTIR spectrum of the PEG-coated triple drug combination encapsulated liposome.
**Figure 13****:** Hydrodynamic size distribution graph of triple drug combination encapsulated liposome coated with PEG.
**Figure 14****:** PH-dependent cumulative drug release graph of carmustine from liposomes at 37°C.
**Figure 15****:** PH-dependent cumulative drug release graph of doxorubicin from liposomes at 37°C.
**Figure 16****:** PH-dependent cumulative drug release graph of curcumin from liposomes at 37°C.
**Figure 17****:** Cumulative release graph of doxorubicin, curcumin and carmustine from liposomes at pH 5.5.
**Figure 18****:** Cumulative release graph of doxorubicin, curcumin and carmustine from liposomes at pH 7.4.

### Detailed Description of the Invention

The invention relates to liposomes containing doxorubicin, carmustine, curcumin drugs and whose surface is coated with 1,2-Distearoyl-Sn-Glycero-3-Phosphoethanolamine-methoxy Polyethylene-Glycol-2000 (DSPE-mPEG). There are dual drug combinations in the previous literature. It is aimed to increase the effectiveness of the treatment by using three drugs with the invention.

The method of preparing the liposome of the invention comprises the following steps:
a) Preparing a pre-emulsion solution in ethanol with phosphodithyl choline: lecithin: cholesterol at a ratio of 0.5-10:0.5-10:0.2-2 by weight.
b) Adding 0.2-8 mL of the drug solution containing 10 µg/mL carmustine, 10 µg/mL doxorubicin and 10 µg/mL curcumin to the lipid mixture dissolved in 0.1-6 mL ethanol.
c) Diluting the obtained pre-emulsion solution with pH 7.4 phosphate buffered saline solution (PBS) at a ratio of 0.2-2:0.5-25 (v/v).
d) Collecting liposomes at the end of the flow 15, wherein the pre-emulsion solution is passed through the microfluidic device at a pressure of 137895 Pa (20.000 psi) and has a diameter of 100- 400 nm.
e) Coating the surface of the obtained liposomes with 10% by weight of 1,2-Distearoyl-Sn-Glycero-3-Phosphoethanolamine-methoxy Polyethylene-Glycol-2000 based on the lipid mixture.

### Liposome Preparation

Liposome preparation was performed by modifying the studies conducted by Huang et al., (2010). Phosphatidylcholine, lecithin, and cholesterol were weighed in certain amounts from phosphatidylcholine, lecithin and cholesterol at a ratio of 0.5-10:0.5-10:0.2-2 by weight, respectively. Subsequently, the total lipid concentration was dissolved in ethanol at 50-60° C with a total lipid concentration of approximately 0.1-6 mL. This ethanolic lipid solution was diluted with pH 7.4 phosphate buffered saline solution (PBS) at a ratio of 0.2-2:0.5-25 (v/v). Vortex was first applied and then kept in the sonic bath in order to ensure the homogeneous distribution of the pre-emulsion solution. This pre-emulsion solution was passed through a microfluidic device (Microfluidizer Processor M-110L) to obtain a liposome of the desired size (in the range of 100-400 nm). An optimization study was carried out to obtain an appropriate size of liposome by applying at 137895 Pa (20.000 psi) pressure and different flow ranges, similar to the study conducted by Lajunen et al., (2014). The hydrodynamic size of liposomes is of great importance in terms of crossing the blood brain barrier (BBB) in the treatment of glioblastoma. It is stated in the literature that it is possible to overcome the blood brain barrier (BBB) with nanoparticles with an average diameter of 100-400 nm (Cetin and Capan, 2004).

Çetin M., Capan Y. (2004). " Beyne Ilaç; Hedeflendirilmesi ", Ankara Eczacilik Fakültesi Dergisi, 33 (4) 287-305.

The samples in the flow 5-20 were collected separately at 137895 Pa (20.000 psi) pressure and hydrodynamic size and polydispersity index (PDI) analyses were performed for the optimization studies. Hydrodynamic size and PDI analyses were performed with a zetasizer (Malvern Nano ZS 90) device. The obtained samples were analyzed for size and PDI. It was found as a result of the analysis that PDI values decreased to 0.15 and 0.12 in the flows 15 and 20, and therefore a more homogeneous distribution was present. The flow 15 samples were determined as optimum in order to be in the appropriate range in terms of size and not to lose energy in the study since aggregation was not detected in the flow 15 sample and no clear difference was observed in the flow 15 and 20 samples.

Zeta potential analysis (Malvern Nano ZS 90 Zetasizer) was performed to determine the load of the liposome sample prepared under optimum conditions according to hydrodynamic and PDI results. Zeta potential, hydrodynamic size, SEM/TEM, FTIR analysis were performed in the liposome structure obtained.

One of the most important phospholipids in liposome structure is phosphatidylcholine (PC). Cholesterol, one of the compounds in the bilayer structure, is generally included in the double layer to increase in vitro and in vivo stability. Cholesterol affects the viscosity of phospholipid double layers and thus reduces their permeability for the drugs captured. Cholesterol above a threshold concentration also reduces the enthalpy change associated with the phase transition of the membrane, causing the liposomes to be less heat sensitive (Sadeghi et al., 2018).

### Preparation and Characterization of Liposome Containing Carmustine, Doxorubicin, and Curcumin

Carmustine (BCNU) was dissolved in ethanol, doxorubicin (DOX) was dissolved in PBS buffer, and curcumin (CUR) was dissolved in acetonitrile. The drug solution containing 10 µg/mL BCNU, 10 µg/mL DOX, and 10 µg/mL CUR in 0.2-8 mL volume was added to the lipid mixture dissolved in 0.1-6 mL ethanol (Lecithin, phosphatidylcholine, cholesterol (0.5-10:0.5-10:0.2-2, w/w)) and the pre-liposome suspension was diluted with pH 7.4 10 mM PBS buffered saline solution at a ratio of 0.2-2:0.5-25 (v/v). Liposome samples were collected by passing the pre-liposome suspension through the microfluidic device 5-25 times at a pressure of 137895 Pa (20.000 psi).

The liposomes encapsulated in BCNU, DOX, and CUR were centrifuged at 12.000 rpm for 30 minutes and the supernatant was separated, 2 washes were performed with PBS buffer under the same conditions. Unbound drugs were removed from the environment with these procedures. Encapsulation efficiency (%) was determined by performing high-performance liquid chromatography (HPLC) (SHIMADZU LC-20AT) analysis in samples containing supernatant and wash water for each drug.

In addition to the determination of the drug content of liposomes prepared by loading BCNU, DOX, and CUR, particle size and zeta potentials were determined using the Zetasizer Ultra (Malvern) device at Ege University Drug Development and Pharmacokinetic Research Application Center (ARGEFAR). In addition, the hydrodynamic size and PDI value and zeta potential of the empty liposome were also analyzed in the ARGEFAR zetasizer Ultra device.

Fourier Transform Infrared (FTIR) analysis will be performed to determine the surface groups of liposomes containing BCNU, DOX, and CUR.

### HPLC Analyses and Validation Studies of Crusting, Doxorubicin, and Curcumin

The analysis of the active substances, carmustine, curcumin, and doxorubicin, was performed by high-pressure liquid chromatography (HPLC) methods during the loading of the drugs into liposomes.

### Carmustine (BCNU) Assay Method

SHIMADZU LC-20A branded High Performance Liquid Chromatography (HPLC) was used to determine the amount of carmustine found in the samples. Inertsil ODS 3-3 (150*4.0) was determined as the analytical column. At a flow rate of 0.8 mL/min, column temperature at 30°C. sample application volume was selected as 20 µL, and for the mobile phase A used, methanol (60:40) containing pH 3.2 0.01 M phosphate buffer was set and for the mobile phase B the detector wavelength was 230 nm. In addition, linearity, selectivity, LOD-LOQ and repeatability parameters were also examined in HPLC validation studies (Dhakane V.D. and Ubale M.B., 2012).

### Doxorubicin (DOX) Assay Method

SHIMADZU LC-20AT branded device was used to determine doxorubicin performed using HPLC. Inertsil ODS 3V (150*4.0) was selected as the column. At a flow rate of 1.25 mL/min, column temperature at 50°C. sample application volume will be 20 µL, and the mobile phase used is Water: Acetonitrile: Tetrahydrofuran (76:24:0.5, v/v/v). pH: 2 (Perchloric Acid). wavelength 480 nm (Excitation), 560 nm (Emission). Then, linearity, selectivity, LOD-LOQ and repeatability parameters were also examined in HPLC validation studies (Alharetha K. et al., 2012). The studies were conducted with standard solutions of doxorubicin. Studies on other parameters will be carried out in a matrix environment.

### Curcumin Assay Method

SHIMADZU LC-20AT (PDA) device was used to determine curcumin to be performed using HPLC. The column used is ACE 5 C18 (250*4.6 mm id). Fixed phase with particle size of 5 microns and pore size of 100 A° shall be used. At a flow rate of 0.75 mL/min, column temperature at 25°C. sample application volume will be 50 µL, and the mobile phase to be used was A: Aqueous (50%) containing 5% M Acetic acid/Water B: Acetonitrile (50%). The wavelength should be 420 nm. In addition, linearity, selectivity, LOD-LOQ and repeatability parameters were also examined in HPLC validation studies (Li J. et al., 2009). The studies were conducted with standard solutions of curcumin. Studies on other parameters will be carried out in a matrix environment.

### Surface Coating, Formulation, Stability Studies of Prepared Liposomes

The surfaces of the liposomes were covered with DSPE-mPEG2000 in order to pass the blood-brain barrier (BBB). Different concentrations of DSPE-mPEG2000 were added to the formed liposomes (1-30% by weight according to the lipid mixture) and left to react at 20-80°C for 1 hour at a constant and slow mixing rate according to the method developed by Mare et al., (Mare et al., 2018). The temperature was quickly brought to 25°C at the end of the time. DSPE-mPEG2000 coated liposomes (MWCO: 12.000-14.000 cellulose membranes) were taken to the dialysis membranes and dialysis was performed for 0.2-5 hours against PBS buffer at the end of the reaction performed in the surface coating section of the liposomes. Thus, drugs that did not participate in the structure and DSPE-mPEG2000 were removed. Zeta potential, hydrodynamic size, and polydispersity index (PDI) measurements of the formed DSPE-mPEG2000 coated liposomes were performed through service procurement at Ege University Drug Development and Pharmacokinetic Research and Application Center (ARGEFAR) on the Zetasizer Ultra (Malvern Nano Ultra, ZSU3305) device. In addition, the last formulation, DSPE-mPEG2000 coated triple drug-loaded liposomes, were dried in the lyophilizer device (Christ 1.2 D Alpha Plus) at the Central Research Test and Analysis Laboratory Application and Research Center (MATAL) of Ege University. FTIR analysis was performed with Perkin Elmer brand UATR Two device for the characterization studies of the dried formulation obtained.

Stability studies of DSPE-mPEG2000 coated liposome formulation containing BCNU-Dox-Cur have been started and are carried out in accordance with the stability guide with the lyophilized product. Samples are stored in colored glass vials at 5±3°C (in the refrigerator), 25±2°C at 60±5% relative humidity and 40±2°C at 75±5% relative humidity. The samples will be checked for 12 months at the time of t=0, that is, at the beginning and at the 1^{st}, 3^{rd}, 6^{th}, 9^{th}, 12th months in the stability study. Zeta potential, particle size and drug content of the formulation are monitored and BCNU-Dox-Cur assays are monitored by HPLC.

### In Vitro Drug Release

Release studies were carried out under continuous stirring at 37°C in two different environments, pH 7.4 PBS buffer and pH 5.5 acetate buffer mimicking the tumor microenvironment. Firstly, the prepared liposomes were transferred to the dialysis membranes (MWCO 12.000-14.000 cellulose membranes). The prepared dialysis membrane was placed in an environment containing 10 mL of buffer and release was initiated. Samples were taken at certain time intervals (1-72 hours) and fresh buffer was added as much as the sample volume and the ambient conditions were kept constant. The amount of drug in the samples taken was determined by HPLC analysis and the % release amounts of the drugs were calculated. HPLC analyses were performed at SHIMADZU LC-20A Ege University Drug Development and Pharmacokinetic Research and Application Center (ARGEFAR).

### CONCLUSIONS AND DISCUSSION

A pre-emulsion was first prepared using phosphatidylcholine, lecithin and cholesterol in the invention. The mean dimensions of liposomes at 137895 Pa (20.000 psi) were found to be 2290±1212 nm without microfluidic administration, 229±295 nm in flow 1, 94±64 nm in flow 3.49±9 nm in flow 10.51±3 nm in flow 15 and 53±3 nm in flow 20. It was determined that liposome sizes decreased and a more monodispersive structure was formed in increased pressure and increased flow passage. Therefore, 137895 Pa (20.000 psi) constant pressure was applied for liposome preparation in the invention.

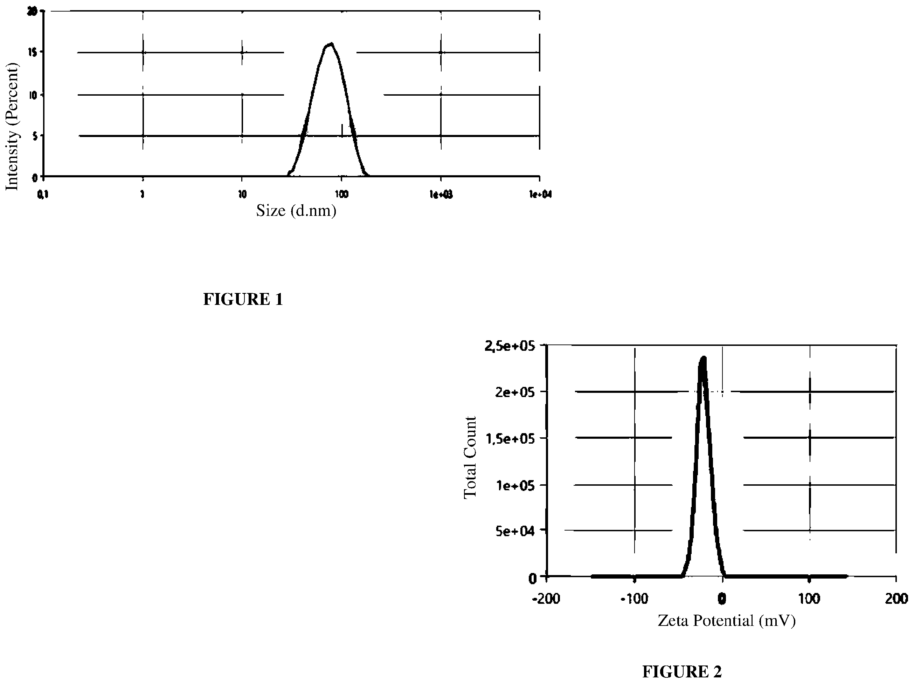

It has been observed in the invention that there are aggregations in the flows 5 and 10 in the experiments carried out at 137895 Pa (20.000 psi) pressure and increasing flow ranges. The hydrodynamic size in flow 5 at 137895 Pa (20.000 psi) is approximately 227±32 nm, the size in flow 10 is approximately 221 ± 14 nm, the size in flow 15 is approximately 228 ± 16 nm, and the size in flow 20 is approximately 219 ± 14 nm. It was observed that the aggregation did not occur in the following flows (flows 15 and 20) and the dimensions remained almost the same. PDI values were approximately 0.34±0.09 in the flow 5 and 0.37±0.04 in the flow 10, 0.15±0.03 in the flow 15 and 0.12±0.03 in the flow 20. PDI values were determined as approximately 0.34 and 0.37 in the flows 5 and 10, respectively, as a result, it was found that PDI values decreased to 0.15 and 0.12 in the flows 15 and 20, and therefore a more homogeneous distribution was present. The flow 15 sample was determined as optimum and given in Figure 1 since aggregation was not detected in the flow 15 sample and no clear difference was observed in the flows 15 and 20 samples.

A zeta potential analysis of the sample prepared in the flow 15 at a pressure of 137895 Pa (20.000 psi) was performed following the hydrodynamic size and PDI values. The measurement result is shown in the graph in Figure 2. The zeta potential value of the empty liposome was measured as approximately -37 ± 2 mV as shown in Figure 2. It is stated that colloidal systems with a zeta potential value greater than +30 mV and less than -30 mV are stable (Vogel et al., 2017). Therefore, considering the zeta potential of the empty liposome sample obtained in the invention, it is thought that it has an appropriate value in terms of both its stability and its ability to pass the BBB.

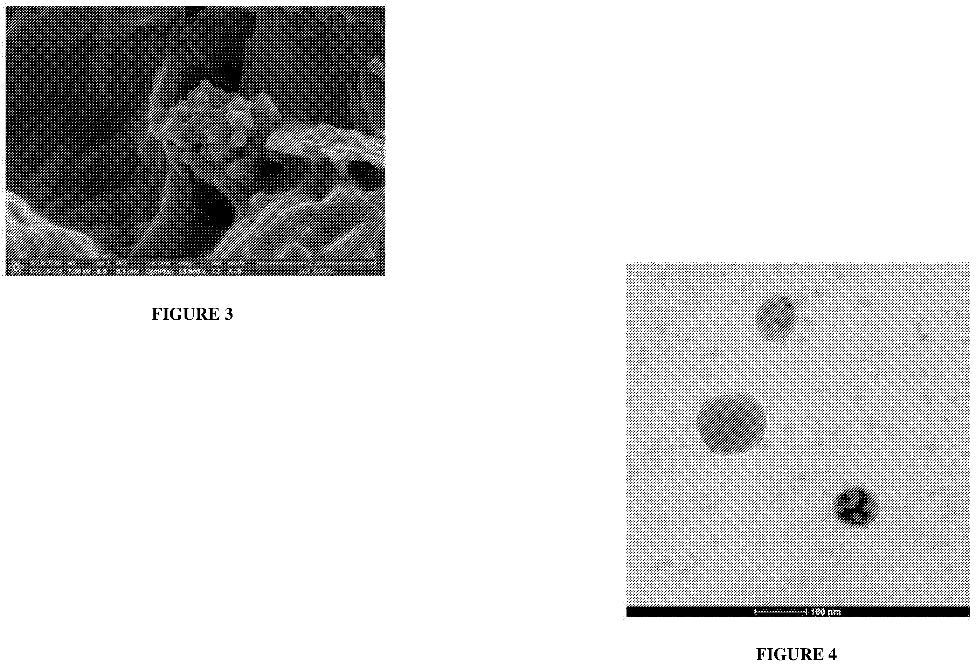

SEM analysis was performed to determine the morphology and size of the liposome sample prepared under optimum conditions. SEM image is given in Figure 3. It is seen as a result of the SEM analysis that the particle sizes in dry form are approximately 120 nm in size and spherical structure. Then, empty liposomes were also analyzed with TEM and their images are given in Figure 4. It is seen as a result of the TEM analysis that the empty liposome sizes are between approximately 154-162 nm. The difference in size in SEM imaging is due to the measurement of liposomes in dry form.

### HPLC Analyses and Validation Studies of Carmustine, Doxorubicin, and Curcumin

### Carmustine Validation Study

**Selectivity:** The chromatograms of the blind sample and the carmustine standard were compared and it was observed that the carmustine did not give any signal at the retention time.

**LOD - LOQ (Specification and Assignment Lower Limits):** LOD and LOQ values were calculated based on signal/noise (SIN) ratios. LOD value was found to be 0.025 µg/mL and LOQ value was found to be 0.082. It was observed that these values met all the studies conducted.

**Linearity:** The calibration curve was formed by plotting the peak area values of 6 different concentrations of the selected standard solution between 0.1 and 10.0 µg/mL against the concentration values. The values of the regression parameters for the curve were calculated with the equation y= ax+b, the values were a=82011, b=1120.4, and R²=0.9999.

Repeatability: The standard solution at a concentration of 1.0 µg/mL was injected into the system 6 times for the repeatability parameter. The standard deviation was calculated as 682.74, RSD %0.813.

### DOX Validation Study

**Selectivity:** The chromatograms of the blind sample and the doxorubicin standard were compared and it was observed that the doxorubicin did not give any signal at the retention time.

**LOD - LOQ (Specification and Assignment Lower Limits):** LOD and LOQ values were calculated based on signal/noise (S/N) ratios. LOD value was found to be 0.0022 µg/mL and LOQ value was found to be 0.0074 µg/mL.

**Linearity:** The calibration curve was formed by plotting the peak area values of 6 different concentrations of the selected standard solution between 0.01 and 2.5 µg/mL against the concentration values. The values of the regression parameters for the curve were calculated with the equation y=ax+b, the values were a=189381, b=-2046.6 and R²=0.994.

**Repeatability:** The standard solution at a concentration of 0.5 µg/mL was injected into the system 6 times for the repeatability parameter. The standard deviation was calculated as 947.60, RSD %1.004.

### Curcumin Validation Study

**Selectivity:** The chromatograms of the blind sample and the curcumin standard dissolved in the mobile phase environment were compared and it was observed that the curcumin did not give any signal at the retention time.

**LOD - LOQ (Specification and Assignment Lower Limits):** LOD and LOQ values were calculated based on signal/noise (S/N) ratios. LOD value was found to be 4.51 ng/mL and LOQ value was found to be 15.04 ng/mL.

**Linearity:** The calibration curve was formed by plotting the peak area values of 6 different concentrations of the selected standard solution between 20 and 1000 ng/mL against the concentration values. The values of the regression parameters for the curve were calculated with the equation y=ax+b, the values were a=500.09, b=-189.19, and R²=1.00.

**Repeatability:** The standard solution at a concentration of 100.0 ng/mL was injected into the system 6 times for the repeatability parameter. The standard deviation was calculated as 178.23, RSD %0.362.

The encapsulation efficiency of liposomes containing Carmustine, Doxorubicin and Curcumin for each drug was determined by calculating the area values of non-encapsulated drugs in supernatants. The results obtained are given in Table 1. It is seen that the active substances are encapsulated in liposomes at high yields when the results are examined. It is seen that there is a slight decrease in yields with the increase in concentration. It has been determined that carmustine and curcumin in lipophilic structure are encapsulated at a much higher rate than doxorubicin.

**Table 1. % encapsulation yields of carmustine, doxorubicin, and curcumin to liposomes at different concentrations.**

| *Concentration (µg*/*mL)* | *Encapsulation yield (%)* | | |
|---|---|---|---|
| | *BCNU* | *DOX* | *CUR* |
| *20* | *96.36* | *65.13* | *89.88* |
| *10* | *99.32* | *67.81* | *92.74* |
| *5* | *100* | *78.12* | *98.15* |
| *2.5* | *100* | *79.60* | *98.55* |

Liposomes containing doxorubicin, carmustine, and curcumin were prepared under optimum microfluidic operating conditions and hydrodynamic size and PDI value were analyzed after encapsulation efficiency calculations in the invention. The hydrodynamic size and PDI values (n=3) of BCNU, DOX, and CUR encapsulated liposomes at varying concentrations are summarized in Table 2.

**Table 2. Hydrodynamic size and PDI values of BCNU, DOX, and CUR encapsulated liposomes at varying concentrations (n=3).**

| *BCNU, DOX, and CUR concentration (µg*/*mL)* | *Hydrodynamic size (nm)* | *PDI value* |
|---|---|---|
| *0 (Empty liposome)* | *110.42±21.31* | *0.235±0.052* |
| *20* | *159.53±2.25* | *0.217±0.014* |
| *10* | *139.30±13.23* | *0.197±0.072* |
| *5* | *134.43±3.07* | *0.267±0.040* |
| *2.5* | *131.08±2.61* | *0.254±0.043* |

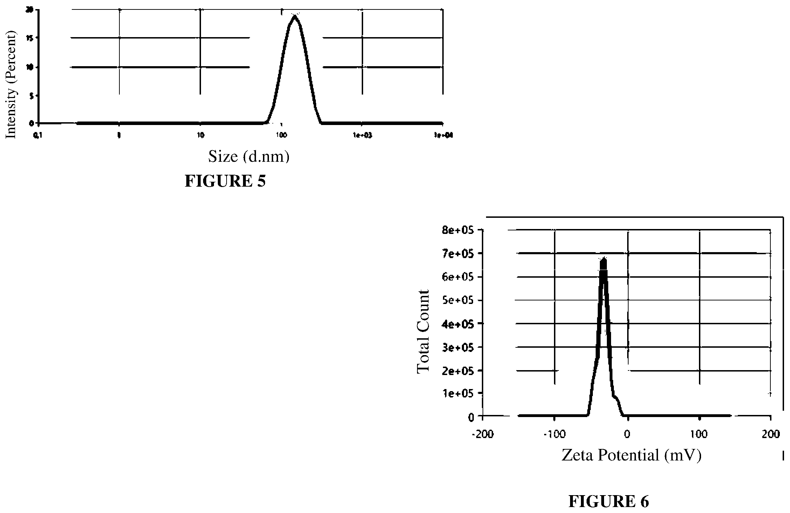

It is seen considering the size values of empty liposomes that there is a slight increase in dimensions with increasing drug concentration. The particle size was determined to be 110 nm in the results of the repeated analysis of empty liposomes in ARGEFAR. The homogeneity of all samples is thought to be good since the PDI values of empty liposomes and drug-loaded liposomes are below 0.3. The hydrodynamic size reached 159 nm and was observed to exceed 150 nm in the triple drug study (20 µg/mL BCNU, DOX, and CUR) at the highest concentration. Since it is predicted that the size of liposomes may increase after the coating of liposomes with DSPE-mPEG2000 in future studies, and considering the encapsulation yields, the optimum drug concentration was determined as 10 µg/mL BCNU, DOX, and CUR, and the hydrodynamic size distribution graph is given in Figure 5.

A zeta potential analysis was performed to determine the net load of BCNU, DOX, and CUR encapsulated liposomes at varying concentrations, and the results are summarized in Table 3. It is stated that colloidal systems with a zeta potential value greater than +30 mV and less than -30 mV are stable (Vogel et al., 2017). It was found in the study that the net load of liposomes obtained at varying concentrations of triple drugs was (-23-(-28 mV). The absolute value of zeta potential decreases due to the increase in drug concentration.

**Table 3. Zeta potential values of BCNU, DOX, and CUR encapsulated liposomes at varying concentrations (n=3).**

| *BCNU, DOX, and CUR concentration (µg*/*mL)* | *Zeta Potential (mV)* |
|---|---|
| *0 (Empty liposome)* | *-30.72±3.18* |
| *20* | *-23.96±1.23* |
| *10* | *-24.17±3.09* |
| *5* | *-25.32±0.67* |
| *2.5* | *-28.43±2.14* |

Thus, considering the zeta potential, HPLC analysis, and size analysis of the liposomes obtained at varying concentrations, it is thought that the triple drug-containing liposomes prepared with BCNU, CUR, and DOX at initial concentrations of 10 µg/mL have an appropriate value in terms of both their stability and their ability to pass the BBB, and the zeta potential graph is given in Figure 6.

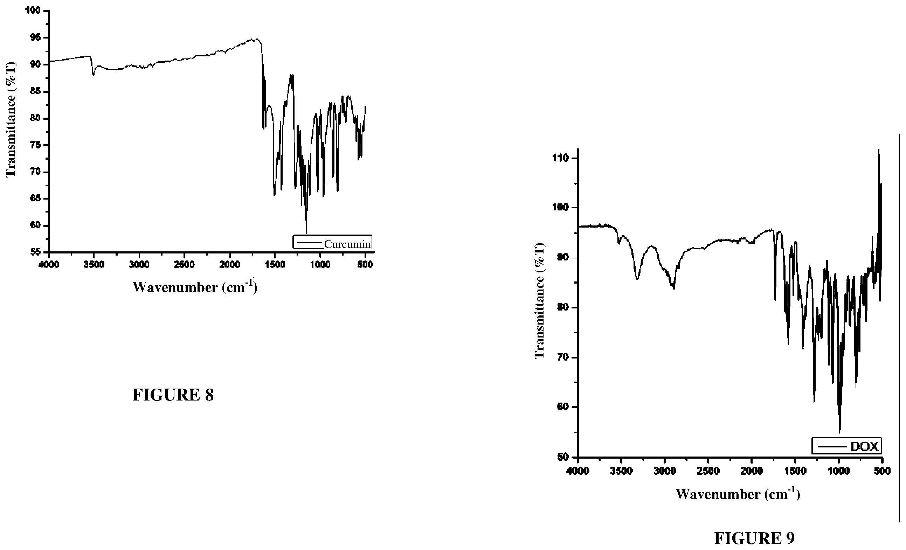

The FTIR spectrum of curcumin is provided in Figure 8. It constitutes the characteristic peak point of the peak-OH phenolic stress band seen at 3340 cm⁻¹. The C=C stretching in the aromatic ring peaks at 1624 cm⁻¹ and C=O is the C=C stretching and olefinic of the symmetric aromatic ring at 1601 cm⁻¹. In addition, the C-H bond is seen at 1425 cm⁻¹ (Xie and Yao, 2020).

The FTIR spectrum of doxorubicin is provided in Figure 9. The peaks at 1281 and 1210 cm⁻¹ according to the FTIR spectrum belong to the C-N stress in the structure of the doxorubicin. In addition, it corresponds to the C=C stretching in the peak aromatic ring at 1411 cm⁻¹. The peaks observed at 1732 cm⁻¹, 3330 cm⁻¹, 2941 cm⁻¹ correspond to the C-O, O-H and C-H bonds found in the structure of doxorubicin, respectively. In addition, the peaks observed at 1620 cm⁻¹ and 1575 cm⁻¹ correspond to the N-H bond in the amine group in the structure of the doxorubicin (Kayal et al., 2010).

### Surface Coating, Formulation, Stability Studies, and Data Analysis of Prepared Liposomes

Hydrodynamic size, zeta potential and PDI values of empty liposome, triple drug combination (BCNU-Dox-Cur) encapsulated liposome, 5-10 and 15% DSPE-MPEG₂₀₀₀ coated liposomes are given in Table 4.

**Table 4. Hydrodynamic size, zeta potential and PDI values of empty liposomes, triple drug encapsulated liposomes, and 5% - 10% - 15% DSPE-mPEG₂₀₀₀ coated liposomes**

| | *Hydrodynamic Size (nm)* | *PDI* | *Zeta Potential (mV)* |
|---|---|---|---|
| *Empty liposome* | *110.42* ± *21.31* | *0.235* ± *0.052* | *-30.72* ± *3.18* |
| *Drug-encapsulated liposome* | *139.30* ± *13.23* | *0.197* ± *0.072* | *-24.17* ± *3.09* |
| *5% PEG coated liposome* | *122.1* ± *7.33* | *0.26* ± *0.01* | *-13.31* ± *1.41* |
| *10% PEG coated liposome* | *120.9* ± *1.65* | *0.27* ± *0.04* | *-15.99* ± *0.65* |
| *15% PEG coated liposome* | *121.7* ± *6.93* | *0.25* ± *0.01* | *-20.17* ± *0.71* |

It is seen when the data in Table 4 are examined that the hydrodynamic dimension value, which increases with drug encapsulation, decreases to around 120 nm with PEG coating. It is thought thanks to the PEG coating that the electrostatic repulsion between the liposomes can reduce the possible and expected aggregation in the liposomes and lead to smaller particle sizes. It is seen when the PDI values of the liposomes in Table 4 are examined that they are around 0.2 for all formulations. This shows that liposomes have a homogeneous size distribution regardless of the involvement of PEG in the liposome structure. It can be said that the dimensions of liposome have a good homogeneity if the PDI value of lipid-based drug carriers such as liposome is <0.3 (Putri et al., 2017). It is observed that the value of -30.72 ± 3.18 mV of the empty liposome decreases with the PEG coating when the zeta potential data in Table 1 are examined. The addition of PEG, which is used as a steric stabilizer, causes a shift in the shear plane of the nanoparticle and thus a decrease in the zeta potential (Heurtault et al., 2003).

Zeta potential is a very important parameter for drug delivery systems that are aimed to cross the blood brain barrier. It is seen that the zeta potential values of PEG coated liposomes are suitable for drug delivery to the brain. The studies were continued by selecting the liposome containing 10% PEG by weight as the optimum according to the lipid mixture when the hydrodynamic size, PDI and zeta potential data were examined.

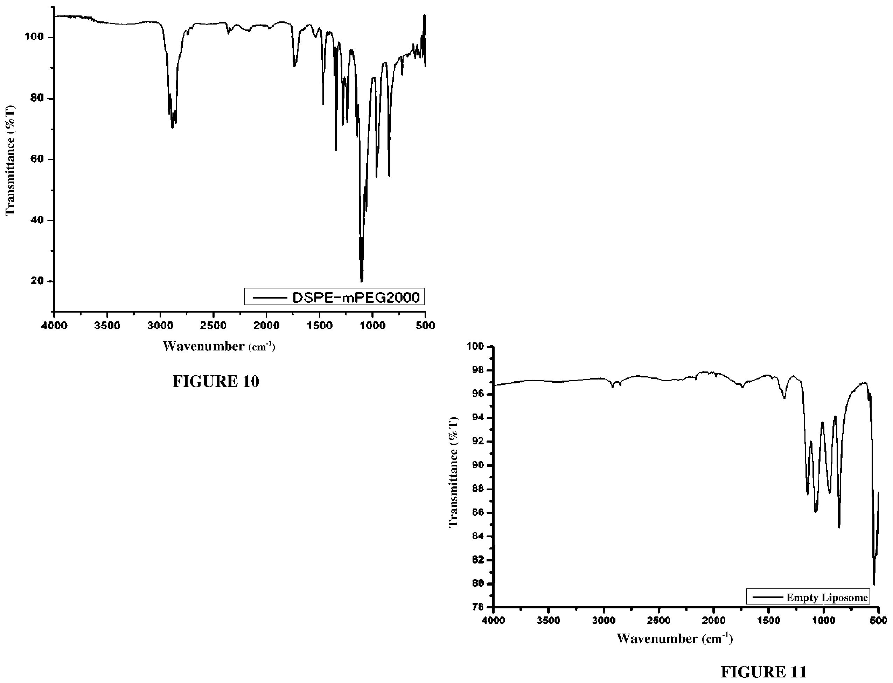

The FTIR spectrum of DSPE-mPEG₂₀₀₀ is given in Figure 10. According to the FTIR spectrum, while it shows a peak of a characteristic carbonyl ketone group at 1738 cm⁻¹, a C-H alkyl stretching peak of DSPE-MPEG₂₀₀₀ at 2922cm⁻¹ and 2848 cm⁻¹ and a characteristic sharp peak of CH₃ at 2885 cm⁻¹ are observed (Abdulla et al., 2010).

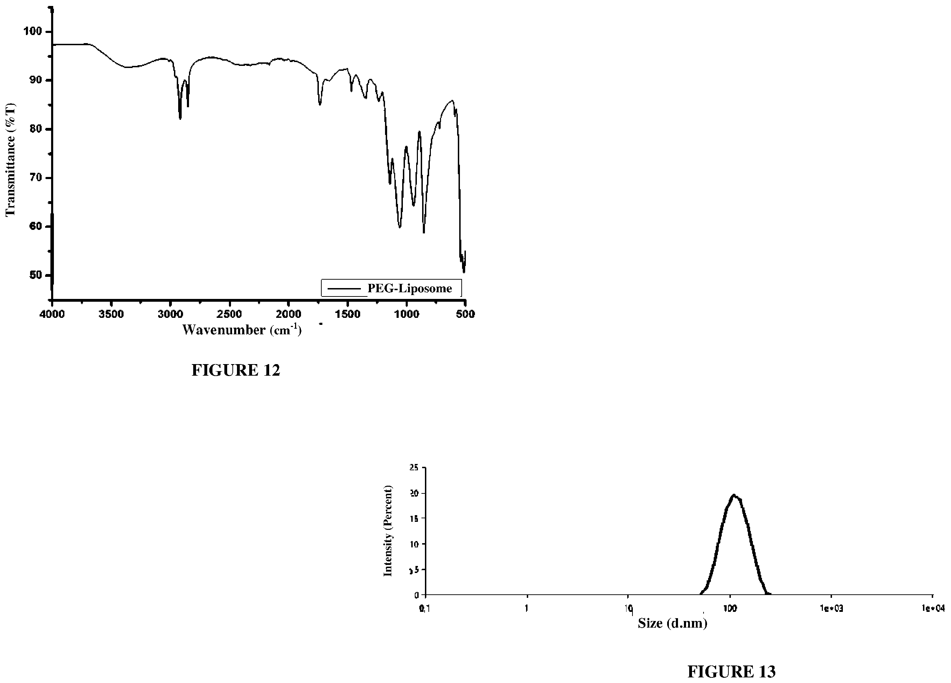

FTIR-transmittance spectra were recorded in ATR (attenuated total reflection) mode between 4000-500 cm⁻¹ wavelengths. The FTIR spectrum of the empty liposome structure is given in Figure 11 and the FTIR spectrum of the triple drug combination encapsulated liposome coated with PEG is given in Figure 12.

In accordance with the structure of the empty liposome, the peak of the stretch vibration of the lipid ester C=O structures is at 1739 cm⁻¹; the IR bands arising from the asymmetric and symmetric stretch vibrations of the lipid-acyl CH₂/CH₃ groups are in the range of 3015-2800 cm⁻¹; the tendency vibration bands of the lipid-acyl CH₂/CH₃ groups are in the range of 1466 and 1358 cm⁻¹. These values are consistent with the IR bands given in the literature (Briuglia et al., 2015, Güler et al., 2016). The P=O stress vibration bands of the phospholipids forming the empty liposome peak at 1238 cm⁻¹ (antisymmetric) and 1072 cm⁻¹ (symmetric). It is known that the ester carbonyl groups in the interface region of the membrane (1760-1700 cm⁻¹) are sensitive to C-O stretch modes, H-bond and polarity changes around them (Güler et al., 2016). Therefore, the IR signal of the empty liposome we created is a wide band peak that has shifted (upshift) to 1739 cm⁻¹ due to the stretch vibrations of the lipid ester C=O structures compared to the individual IR band positions (1736 cm⁻¹) of the lipids (lecithin, phosphatidylcholine and cholesterol) in the empty liposome content. This shows that the C=O molecules of the carbonyl groups in the empty liposome structure are located in the interface region of the liposome and make less H-bonding (less polar). This indicates that liposomes are formed.

Similarly, the IR signals of the lipid-acyl CH₂/CH₃ groups in the empty liposome structure were as low as the number of 1-2 cm⁻¹ waves, indicating that the lipid-acyl CH₂/CH₃ groups had a more regular structure within the liposome. The antisymmetric and symmetric O-P-O stretch vibration bands of the phospholipids forming the empty liposome appear as a wide peripheral band at 1238 cm⁻¹ and 1072 cm⁻¹, respectively. This indicates that the anionic phospholipid head groups of the empty liposome form an H-bond and contain more than one H-linked molecular group. Therefore, it is clearly understood that the anionic phospholipid head groups in the empty liposome structure are the structures that are exposed to the solvent around the liposome, the C=O molecules of the carbonyl groups are in the interface region of the liposome, and the lipid-acyl CH₂/CH groups have a more regular structure within the liposome.

When the PEG-coated medicated liposome FTIR spectrum (Figure 12) is examined, the strong IR bands caused by OH stretch vibrations in the chemical structure of curcumin, doxorubicin and cholesterol are in the range of 3500-3200 cm⁻¹ (1652 cm⁻¹: OH bending vibrations). However, a very weak OH signal (both stretch and bending bands) is received in the empty liposome spectrum. This indicates that the drugs participate in the liposome structure. Again, when compared with the empty liposome FTIR spectrum, in the PEG-coated medicated liposome spectrum (in the range of 3015-2800 cm⁻¹), each IR band mostly caused by the asymmetric and symmetric stretch vibrations of the CH₂/CH₃ groups shifts to the lower wave number (down shifting). This clearly shows that the lipid-acyl CH₂/CH₃ groups have a more regular structure within the liposome in the presence of the drug and encapsulation occurs.

While the IR signal due to the stretch vibrations of the C=O structures is at 1739 (empty liposome), 1732 (doxo), 1713 (carmustine) and 1737.8 (PEG) cm⁻¹, it is a wide band peak that has shifted to 1737.5 cm⁻¹ (downshift) in the PEG coated drugged liposome spectrum. In the range of 1500-1300 cm⁻¹, signal increases and band shifts are observed in the peaks mostly caused by the bending vibrations of the CH₂/CH₃ groups, and in the P=O stretching vibration bands of the phospholipids at 1250-1200 cm⁻¹ (anti-symmetric) and 1060 cm⁻¹ (symmetric stretching, shifted to 1072-1058 cm⁻¹). In the PEG-coated drugged liposome spectrum, the IR signal caused by curcumin C-O-C vibrations at 1277 cm⁻¹ is also noteworthy.

Therefore, it is clearly understood that the liposome lipid acyl structures are more regular in the presence of drugs and PEG and encapsulation occurs.

The hydrodynamic size graph of the liposome selected as the optimum liposome containing 10% PEG by weight according to the lipid mixture is given in Figure 13.

### In Vitro Drug Release

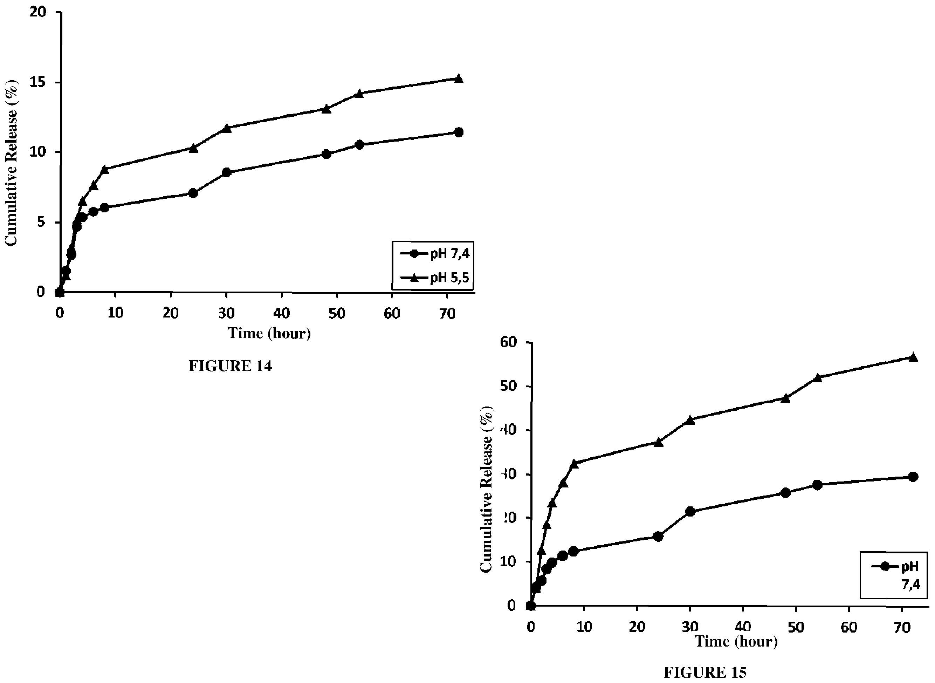

Drug release studies of Peg coated liposome formulation containing triple drug (BCNU-Dox-Cur) were carried out at 37°C and under 300 rpm mixing rate by dialysis bag method in pH 7.4 and pH 5.5 environments. The results of the pH-dependent release study of drugs from liposomes are given in Figure 14-18.

It was observed that the release of all drugs was higher at pH 5.5 when the obtained data were examined. Cancerous cells face an oxygen deficiency called hypoxia, which causes a pH drop in the tumor microenvironment. Therefore, it is desirable to have high release percentages at pH 5.5, which is similar to the tumor microenvironment.

Figure 14 shows the pH-dependent cumulative drug release graph of carmustine from liposomes at 37°C. BCNU released from liposomes at the end of 72 hours is 15.36% at pH 5.5 and 49% at pH 7.4 when Figure 14 is examined.

Figure 15 shows the pH-dependent cumulative drug release graph of doxorubicin from liposomes at 37°C. It was seen that the amount of DOX released at pH 7.4 was 29.68% at the end of 72 hours and this rate reached 56.96% at pH 5.5 when Figure 15 was examined.

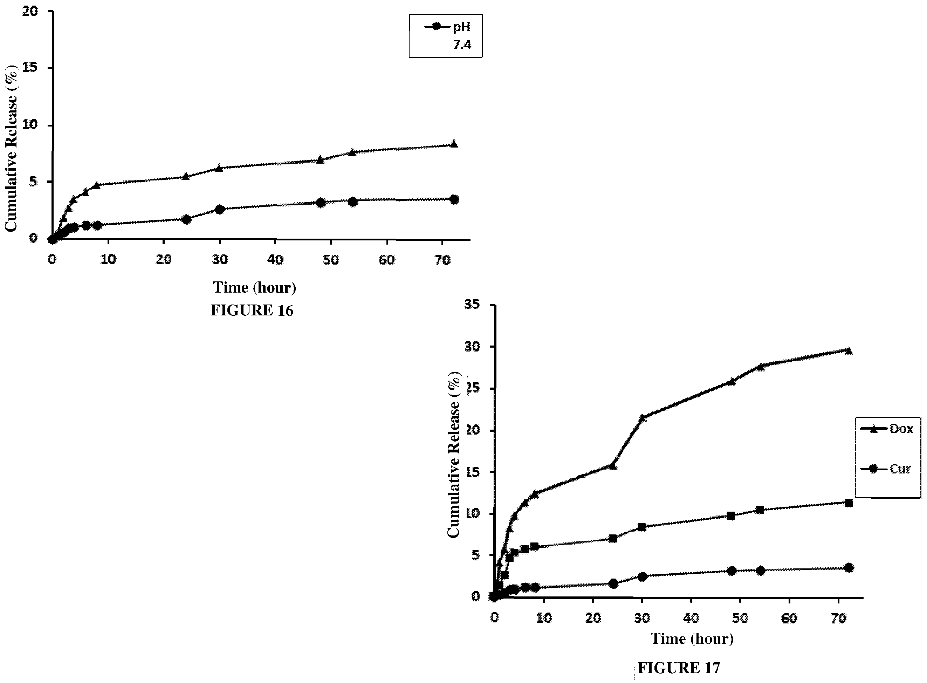

Figure 16 shows the pH-dependent cumulative drug release graph of curcumin from liposomes at 37°C. It is seen that the Cur release from liposomes is 8.42% at pH 5.5 and 3.59% at pH 7.4 at the end of 72 hours when Figure 16 is examined.

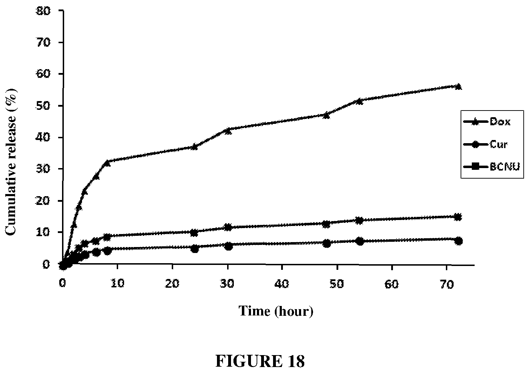

Figure 17 shows the cumulative release graph of doxorubicin, curcumin and carmustine from liposomes at pH 5.5. It is seen that Dox has the best release percentage by being released at the end of 48 hours when Figure 17 is examined. Doxorubicin has high solubility at low pH due to its structure. Therefore, it is likely to be released at a high rate at low pH (Nguyen et al., 2013). The lower release of Cur compared to Dox and BCNU may be due to the hydrophobic interaction between Cur and the double layer, the stronger the interaction, the slower the release of curcumin from liposomes (Li et al., 2018). Figure 18 shows the cumulative release graph of doxorubicin, curcumin and carmustine from liposomes at pH 7.4. It is seen that the lowest cumulative release percentage belongs to Cur again when Figure 18 is examined, Dox, which has a high release at low pH, is almost unaffected by a neutral pH (Nguyen et al., 2013).

The main purpose of drug delivery systems is to provide a continuous and long-term treatment by providing controlled and slow drug release in the cancerous area. It is clear that drug release from liposomes is higher in an acidic environment similar to the tumor microenvironment compared to the physiological pH considering the drug release data. It is thought in this case that liposomes may act in cancerous regions without causing undesirable side effects in healthy tissues and may cause a controlled and long-term drug release in these regions.

## Claims

1. A liposome, **characterized in that** it comprises doxorubicin, carmustine and curcumin drugs and its surface is coated with 1,2-Distearoyl-Sn-Glycero-3-Phosphoethanolamine-methoxy Polyethylene-Glycol-2000 (DSPE-mPEG).

2. A method of preparing the liposome according to claim 1, **characterized in that** it comprises the process steps of;
a) Preparing a pre-emulsion solution in ethanol with phosphodithyl choline: lecithin: cholesterol at a ratio of 0.5-10:0.5-10:0.2-2 by weight.
b) Adding 0.2-8 mL of the drug solution containing 10 µg/mL Carmustine, 10 µg/mL doxorubicin and 10 µg/mL curcumin to the lipid mixture dissolved in 0.1-6 mL of ethanol.
c) Diluting the obtained pre-emulsion solution with pH 7.4 phosphate buffered saline solution (PBS) at a ratio of 0.2-2:0.5-25 (v/v).
d) Collecting liposomes at the end of the flow 15, wherein the pre-emulsion solution is passed through the microfluidic device at a pressure of 20.000 psi and has a diameter of 100-400 nm.
e) Coating the surface of the obtained liposomes with 10% by weight of 1,2-Distearoyl-Sn-Glycero-3-Phosphoethanolamine-methoxy Polyethylene-Glycol-2000 based on the lipid mixture.

3. A liposome according to claim 1, **characterized in that** the hydrodynamic size is in the range of 100-400 nm.

## Patentansprüche

1. Liposom, **dadurch gekennzeichnet, dass** es die Wirkstoffe Doxorubicin, Carmustin und Curcumin umfasst und seine Oberfläche mit 1,2-Distearoyl-sn-glycero-3-phosphoethanolamin-methoxypolyethylenglycol-2000 (DSPE-mPEG) beschichtet ist.

2. Verfahren zum Herstellen des Liposoms nach Anspruch 1,
**dadurch gekennzeichnet, dass** es die Prozessschritte umfasst;
a) Herstellen einer Voremulsionslösung in Ethanol mit Phosphatidylcholin: Lecithin: Cholesterin in einem Gewichtsverhältnis von 0,5-10 : 0,5-10 : 0,2-2.
b) Zugeben von 0,2-8 mL der Wirkstofflösung, die 10 µg/mL Carmustin, 10 µg/mL Doxorubicin und 10 µg/mL Curcumin enthält, zu der in 0,1-6 mL Ethanol gelösten Lipidmischung.
c) Verdünnen der erhaltenen Voremulsionslösung mit phosphatgepufferter Salzlösung (PBS) mit einem pH-Wert von 7,4 in einem Verhältnis von 0,2-2 : 0,5-25 (v/v).
d) Sammeln von Liposomen an dem Ende des Flusses 15, wobei die Voremulsionslösung bei einem Druck von 20.000 psi durch die mikrofluidische Vorrichtung geleitet wird und einen Durchmesser von 100-400 nm aufweist.
e) Beschichten der Oberfläche der erhaltenen Liposomen mit 10 Gew.-% 1,2-Distearoyl-sn-glycero-3-phosphoethanolamin-methoxypolyethylenglycol-2000, bezogen auf die Lipidmischung.

3. Liposom nach Anspruch 1, **dadurch gekennzeichnet, dass** die hydrodynamische Größe in dem Bereich von 100-400 nm liegt.

## Revendications

1. Liposome, **caractérisé en ce qu'**il comprend les médicaments doxorubicine, carmustine et curcumine et dont la surface est revêtue de 1,2-distéaroyl-sn-glycéro-3-phosphoéthanolamine-méthoxy polyéthylène-glycol-2000 (DSPE-mPEG).

2. Procédé de préparation du liposome selon la revendication 1,
**caractérisé en ce qu'**il comprend les étapes de procédé de ;
a) Préparation d'une solution de pré-émulsion dans de l'éthanol avec de la phosphodithyl choline : lécithine : cholestérol selon un rapport en poids de 0,5-10:0,5-10:0,2-2.
b) Ajout de 0,2 à 8 mL de la solution médicamenteuse contenant 10 µg/mL de carmustine, 10 µg/mL de doxorubicine et 10 µg/mL de curcumine au mélange lipidique dissous dans 0,1 à 6 mL d'éthanol.
c) Diluer la solution de pré-émulsion obtenue avec une solution saline tamponnée au phosphate (PBS) à pH 7,4 à un rapport de 0,2-2:0,5-25 (en pourcentage volumique).
d) Collecter des liposomes à la fin de l'écoulement 15, dans lequel la solution de pré-émulsion est passée à travers le dispositif microfluidique à une pression de 20 000 psi et a un diamètre de 100 à 400 nm.
e) Revêtir la surface des liposomes obtenus avec 10 % en poids de 1,2-distéaroyl-sn-glycéro-3-phosphoéthanolamine-méthoxy polyéthylène-glycol-2 000 par rapport au mélange lipidique.

3. Liposome selon la revendication 1, **caractérisé en ce que** la taille hydrodynamique est dans la plage de 100 à 400 nm.
